(19) Europäisches Patentamt

European Patent Office

Office européen des brevets

(11) Publication number: **0 125 879**
**B1**

(12) **EUROPEAN PATENT SPECIFICATION**

(45) Date of publication of patent specification: **01.02.89**

(21) Application number: **84303136.0**

(22) Date of filing: **09.05.84**

(51) Int. Cl.⁴: **H 05 G 1/06,** A 61 B 6/02, H 01 R 39/46

(54) Integrated CT scanner gantry.

(30) Priority: **12.05.83 US 494104**

(43) Date of publication of application:
**21.11.84 Bulletin 84/47**

(45) Publication of the grant of the patent:
**01.02.89 Bulletin 89/05**

(84) Designated Contracting States:
**DE GB NL**

(56) References cited:
**EP-A-0 039 994**
**FR-A-2 333 237**
**GB-A-2 000 738**
**GB-A-2 061 028**
**US-A-4 201 430**
**US-A-4 366 577**

(73) Proprietor: **KABUSHIKI KAISHA TOSHIBA**
**72, Horikawa-cho Saiwai-ku**
**Kawasaki-shi Kanagawa-ken 210 (JP)**

(72) Inventor: **Bernardi, Richard Thomas**
**100 East Clarendon Street**
**Prospect Heights Illinois (US)**

(74) Representative: **Shindler, Nigel et al**
**BATCHELLOR, KIRK & EYLES 2 Pear Tree Court**
**Farringdon Road**
**London EC1R 0DS (GB)**

Courier Press, Leamington Spa, England.

## Description

The present invention relates to the support structure, or gantry, for an x-ray computed tomography (CT) scanner or the like.

X-ray CT scanners are known which comprise an x-ray assembly mounted on a rotatable support. Such rotatable supports typically comprise a fixed stator and a rotor rotatably mounted thereon by means of a ring bearing. The stator and rotor have a hollow bore to permit the whole body of a patient to lie along the axis of rotation of the rotor. An x-ray source and x-ray optics, including a shutter, wedges, and a collimator, are all individually mounted to the rotor and aligned with respect to one another.

The x-ray source requires access to high voltage. High voltage cables may be employed to connect a stationary high voltage source to the rotatable x-ray source. However, the cables then inhibit the degree by which the x-ray source may be rotated about the patient, and preclude continuous rotation of the x-ray source.

To provide continuous rotation of the x-ray source, known gantry assemblies employ the use of a standard high voltage slip ring assembly. Such a slip ring assembly may have its own rotor and stator structures which are supported on its own ring bearing. Known slip ring configurations require liquid or gas high voltage insulation and attendant seals. These seals are subject to repeated failure, requiring easy access to the seals and/or the ability to easily replace the entire slip ring assembly. Still further, known slip rings require electrical connection in a manner which results in close proximity crossing of high voltage wiring of one polarity with ring electrodes of the opposite polarity. Such crossings have the tendency to result in high voltage breakdown.

Such known slip ring assemblies are constructed with a small diameter in order to minimise cost and in order to avoid disadvantages typically incumbent in large diameter slip rings. The diameter, accordingly, is typically too small to encircle a patient. The slip ring assembly is therefore, located at one end of the gantry assembly, and an arm is used to connect the slip ring assembly to the rotor of the x-ray assembly. The resultant gantry assembly forms a closed-bore into which the patient must be inserted, precluding access to the patient and intensifying patient concern. Moreover, such prior art gantry assemblies have minimal, if any, ability to tilt the plane in which the x-ray source rotates.

One type of gantry assembly which does have the capability of being tilted to a limited degree about an axis parallel to the floor is shown in GB—A 20 61 028. In order to increase the possible angle of tilt it is also known from GB—A 20 00 738 to form the stator in a truncated conical shape.

A gantry assembly can also be constructed with a large diameter slip ring to avoid the disadvantage of a close-bore configuration. In such a device, the x-ray assembly and slip ring assembly could be positioned side-by-side with fins or the like physically interconnecting the two assemblies. Once again, however, the ability to tilt the plane of the x-ray source is limited. To maximise the degree of possible tilt, the internal diameter of both the x-ray and slip ring assemblies must be increased, but with incumbent increase in bulk cost and complexity of the resultant gantry assembly. More importantly, for given rotor rotation speeds, surface speeds increase with larger diameter assemblies, which increases the probability of seal failure and the failure of other components. In known gantry assemblies, cost is further increased by utilising separating ring bearing assemblies, stator structures and rotor structure, for both the x-ray assembly and the slip ring assembly.

Another known form of gantry assembly is shown in European patent application No. 00 39 994 and comprises:

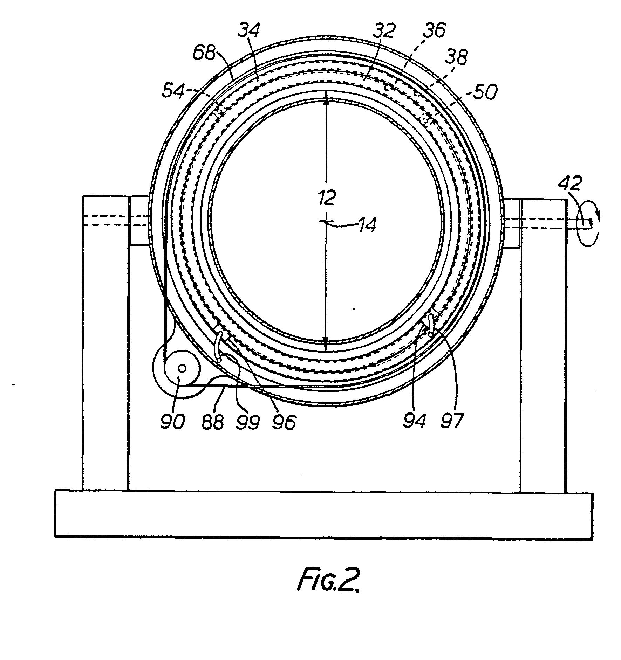

(a) a stator 14 having first and second cylindrical sections of different diameters, said first section having a smaller diameter than said second section and having a diameter sufficient to encircle a patient positioned along an axis of rotation (Figure 2) and a first annular plate section lying in a plane perpendicular to said axis of rotation (Figure 2);

(b) a rotor 18 having a cylindrical section aligned with and coaxial to said first and second cylindrical sections of said stator to form an annular recess with said first plate section of said stator closing off one side of said recess and an annular plate section 20 lying in a plane perpendicular to said axis of rotation, said annular plate section extending from one side of said cylindrical section of said rotor to close off the other side of said recess (Figure 2);

(c) a bearing assembly 34 located between said stator and said rotor to rotatably support said rotor on said stator, (Figure 2);

(d) first 28—31, 80, 66—68 and second 32 slip ring means mounted in part on said stator and in part on said rotor and extending into said recess, for effecting high voltage connection from said stator to said rotor while permitting continuous rotation of said rotor, (Figures 2, 5).

Accordingly, the present invention seeks to provide a gantry assembly which allows for continuous rotation of an x-ray source in an economical and efficient manner, and has an open bore configuration with a minimal gantry cross-sectional area and overall volume, thus permitting unrestricted patient access.

The present invention further seeks to provide a continuous rotation gantry assembly which readily permits tilting of the plane in which the x-ray source rotates, and in which the x-ray source and x-ray optics may readily be aligned at a location remote from the gantry assembly.

The present invention also seeks to provide a continuous rotation gantry assembly which minimises the likelihood of high voltage breakdown.

The present invention also seeks to provide a continuous rotation gantry assembly containing liquid or gas insulation seals which are not subject to repeated failure and which are easily accessible for replacement in the unlikely event of failure.

Accordingly the present invention provides an integrated gantry assembly, for an x-ray scanner comprising:

an integrated gantry assembly for an x-ray scanner comprising:

a stator (10) having an axial bore of sufficient size to encircle a patient positioned along the axis (14);

a rotor (16) havbing an axial bore of sufficient size to encircle a patient positioned along said axis;

a bearing assembly (18) located between said stator (10) and said rotor (16) so as to align said stator (10) and rotor (16) coaxially and to support said rotor (10) rotatably on said stator (10);

slip ring means (44 and 48) mounted in part on said stator (10) and in part on said rotor (16) and located between said rotor (16) and stator (10) for effecting high voltage connection from said stator (10) to said rotor (16) while permitting continuous rotation of said rotor (16); and

X-ray means (74) mounted onto and supported solely by said rotor (16) and defining an exposure position for said patient; characterised in that:

said stator (10) and rotor (16) define between them first and second annular recesses (36 and 38), said first recess (36) being positioned axially closer to said exposure position and having a smaller diameter than said second recess (38), one of said recesses (36 and 38) housing a negative electrode (44 or 48) of said slip ring (44 and 48) means and the other of said recess (36 or 38) housing a positive electrode (44 or 48) of said slip ring means (44 and 48).

Preferably, the inside surface of the stator which faces the patient generally lies along the surface of a truncated cone and the stator and rotor define between them first and second annular recesses. The first recess preferably has a smaller diameter than the diameter of the second recess and the recesses are offset one from the other along the longitudinal axes of the stator and rotor to permit one of the recesses to effectively house a negative electrode of the slip ring means and the other of the recesses to effectively house a positive electrode of the slip ring means without risk of breakdown or crossover between the negative and positive electrode.

In a preferred embodiment of the invention, the stator and rotor have a cross section formed as a series of matching steps with a horizontal portion of at least two of the steps of the stator separated from an aligned horizontal portion of the steps of the rotor to form the two electrically and physically separated recesses mentioned above. It is also preferred that first and second seals be pancaked between the steps of the rotor and stator, with one seal located on each side of the abovementioned recesses. Further it is also preferable that the seals can be located by a respective seal retainer, and that the seal retainers be adjustably positionable to pre-load the seals, thereby controlling the amount of friction introduced to the gantry assembly by the seals and the amount of torque required to rotate the rotor of the gantry assembly.

Electrical connections are made to the recesses through vertical portions of the steps of the stator which define the recesses, wherein the axes of the electrical connectors are off-set from one another and parallel to the longitudinal axes of the rotor and stator. This orientation avoids the crossover incumbent in prior art slip rings and thereby eliminates the possibility of high voltage breakdown due to such crossover.

Preferably the x-ray means of the gantry assembly of the subject invention comprises: a bracket removably attached to the rotor; an x-ray source attached to the bracket; and x-ray optics also attached to the bracket, whereby the x-ray source and x-ray optics can be aligned on the bracket remote from the rotor and stator.

One embodiment of the invention will now be described by way of example with reference to the accompanying drawings in which:

Figure 1 is a semi-diagrammatic cross-sectional view of one section of the rotor and stator of a gantry assembly; and

Figure 2 is another semi-diagrammatic cross-sectional view of a gantry assembly similar to that of Figure 1.

In the drawings, there is illustrated a stator 10 having an opening 12 along a longitudinal axis 14 thereof of sufficient size to permit stator 10 to encircle a patient positioned along axis 14. Stator 10, being opened along axis 14, allows access to a patient from both sides of stator 10. A rotor 16 is concentrically mounted with stator 10 and also has an opening 12 along the longitudinal axis 14. Rotor 16, accordingly, also provides access to the patient from both sides of the rotor. A bearing assembly 18 is located between stator 10 and rotor 16 so as to align and support rotor 16 rotatably on stator 18.

As illustrated, stator 10 has a stepped cylindrical cross-section comprising annular cylindrical sections 18, 20, 22 alternating with annular plate sections 24, 26. The first and second cylindrical sections 20, 22 are coaxial with axis 14 and offset from one another along axis 14, the first section 20 having a smaller diameter than the second section 22. However, the first section 20 has a diameter which is sufficient to encircle a patient positioned along axis 14.

The first and second annular plate sections 24 and 26 lie in planes perpendicular to axis 14. The first annular plate section 24 extends the first and second annular sections 20 and 22, and the second annular plate section 26 extends radially outwardly from the other edge of cylindrical section 22 in a direction away from axis 14.

Rotor 16 also has a stepped cylindrical cross-section and comprises annular cylindrical sections 28, 30, alternating with annular plate sections 32 and 34. First and second cylindrical sections 28 and 30 of rotor 16 are respectively aligned and coaxial with first and second cylindrical sections 20 and 22 of stator 10. The diameter of the first rotor section 28 is greater than the diameter of first stator section 20, thereby forming a first annular recess 36 therebetween. Likewise, second rotor section 30 has a larger diameter than the second stator section 22, thereby forming a second annular recess 38 therebetween, with first annular plate section 24 of stator 10 closing the outer end of recess 36 and with second annular plate section 26 of stator 10 closing off the outer end of recess 38.

The first and second annular plate sections 32, 34 of rotor 16 lie in planes perpendicular to axis 14. The first annular plate section 32 extends radially inwardly from cylindrical section 28 toward axis 14 to close off the inner end of recess 36, while second annular plate section 34 extends radially outwardly from cylindrical section 28 of rotor 16 away from axis 14 to one edge of the second cylindrical section 30 of rotor 16 to close off the other end of recess 38.

Accordingly, the stator 10 and rotor 16 comprises a series of coaxial cylinders with cylindrical portions of the stator (sections 20, 22) separated from the corresponding portions of the rotor (sections 28, 30) to form two electrically and physically separated annular recesses 36 and 38 therebetween.

As a result of the construction of stator 10 in this way, the inside surface of stator 10 which faces a patient lying along axis 14 generally lies in the surface of a truncated cone illustrated by dotted line 40. Accordingly, stator 10 may be tilted about an axis 42 by an angle which approaches an angle formed by the intersection of axis 14 and dotted line 40. With ring bearing assembly 18 located as illustrated along first cylindrical section 20, in combination with the use of the illustrated step-like configuration of stator 10, the degree of tilting permitted around axis 42 is maximised while the diameter of bearing assembly 18 is minimised.

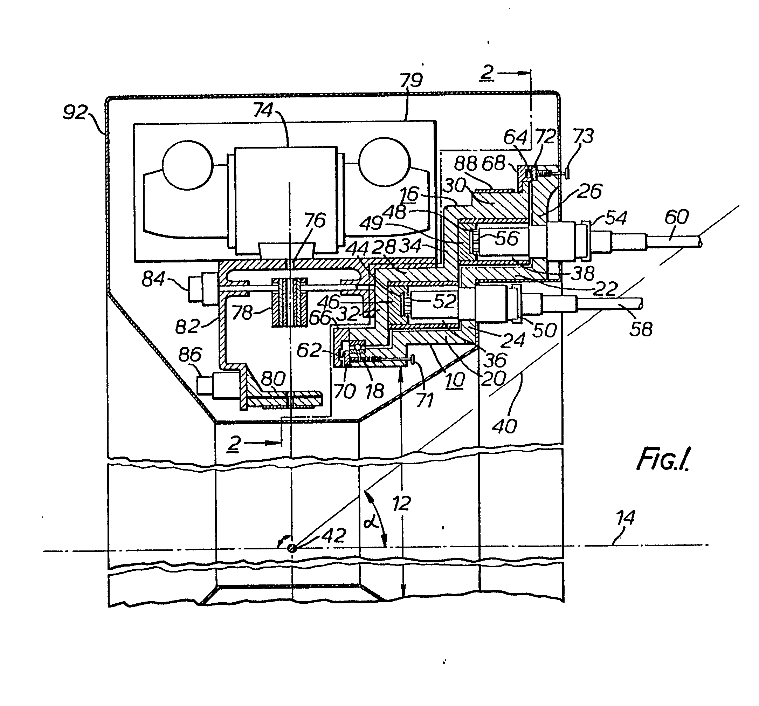

A slip ring is mounted in part on the stator of the gantry assembly and in part on the rotor of the gantry assembly to effect high voltage connection from the stator to the rotor while permitting continuous rotation of the rotor. More specifically, as illustratively shown in Figure 1, a first pancake electrode 44 in the form of a ring is mounted on first annular plate section 32 by means of an electrical insulator 46. A second pancake electrode 48 in the form of a ring is mounted to second annular plate section 34 of rotor 16 by means of an electrical insulator 49. A stator connector and brush block assembly 50 extends through first plate section 24 of stator 10 into recess 36. Assembly 50 includes brushes 52 which contact ring 44 within recess 36.

In a similar manner, stator connector and brush block assembly 54 extends through second annular plate section 26 of stator 10 into recess 38. Brushes 56 on assembly 54 contact electrode ring 48. Although shown in Figure 1 to be immediately adjacent to one another in Figure 1, assemblies 50, 54 are preferably circumferentially spaced apart from one another as shown in Figure 2.

Rotor high voltage connectors 96 and 94 are shown in Figure 2 extending through annular plates 34 and 32, respectively, and are separated by some degrees from one another. The axes of assemblies 96 and 94 are also parallel to axis 14.

As a consequence of the matching step configurations of stator 10 and rotor 16 and the resultant formation of annular recesses 36 and 38, in combination with the use of pancake ring electrodes 44 and 48, the axes of assemblies 50, 54 are off-set from one another and parallel to axis 14. The high voltage electric cables 58, 60 are connected to assemblies 50, 54 which in turn are connected by insulated conductors to the slip ring brushes 52, 56 without any kinks, bends or twists. This configuration avoids having any poorly insulated electrical conductors run adjacent, or cross directly over rings of any opposite polarity electrodes. A separation is instead provided between any such conductors and rings of opposite polarity electrodes, which avoids high voltage breakdown that can occur in standard large diameter prior art high voltage slip ring devices not having such separation. In a similar manner high voltage electric cables 97 and 99 are connected to assemblies 94 and 96, as shown in Figure 2, which in turn are connected by insulated conductors to rings 44 and 48 without any kinks, bends or twists. This configuration avoids having any poorly insulated electrical conductors run adjacent, or cross directly over rings of any opposite polarity electrodes. A separation is instead provided between any such electrical conductors and rings of opposite polarity electrodes which avoids high voltage breakdown that can occur in standard large diameter prior art high voltage slip ring devices not having such separation.

It is preferable that high voltage insulation in the form of an inert gas or liquid be introduced and held within recesses 36 and 38. To seal such liquid or gas within recesses 36 and 38, there is provided front slip ring seal 62 located on one side of recesses 36 and 38 and rear slip ring seal 64 located on the other side of recesses 36 and 38. More specifically, there are provided seal retainer rings 66, 68 one coupled to the front end of rotor 16 to house seal 62 and one coupled to the rear end of rotor 16 to house seal 64. Seal running surface 70 is coupled to the front end of stator 10 and mates against front slip ring 62. Seal running surface 72 is coupled to the front side of second disc section 26 of stator 10 and mates against ring seal 64. Retainers 66, 68 and running surfaces 70, 72 are readily accessible and may easily be removed and/or adjusted to either replace ring seals 62, 64 or adjust the preload to which these seals are exposed. Bolts 71 and 73 are illustrated in Figure 1 as being adjustably attached to surfaces 70, 72 to readily adjust the location of these surfaces and, hence, adjust the preload to seals 62, 64. Accordingly, the life expectancy of slip ring seals 62, 64 is greatly increased over known prior art configurations. As shown in Figure 1, an x-ray assembly comprising an x-ray source 74, x-ray optics including a shutter assembly 76, wedge assembly 78, and a pre-patient collimator assembly 80 are all mounted on an x-ray mounting bracket 82. X-ray mounting bracket 82 is removably attachable to rotor 16, and when removed from rotor 16, bracket 82 permits alignment of the x-ray optics with x-ray source 74 remote from rotor 16 and stator 10. Moreover, with x-ray mounting bracket 82 affixed to rotor 16, either at first annular plate section 32 and/or at first cylindrical section 28, the x-ray source envelope 79 which defines the permissible location of x-ray source 74, may extend over first cylindrical section 28 thereby minimising the overall width of the resultant assembly.

Figure 1 also shows a motor 84 to control positioning of wedge assembly 78, a motor 86 to control the opening of collimator assembly 80, a drive belt 88 on second cylindrical band section 30 which is coupled to a motor 90 (Figure 2) which in turn is mounted on stator 16. A cover 92 is shown to encase the entire integrated gantry assembly.

The integrated gantry assembly of the illustrated embodiment accordingly provides for continuous rotation of an x-ray source through use of a high voltage slip ring to transmit high voltage power from stationary to rotating members. Unlike prior art slip ring assemblies, however, which are physically independent from the structure which supports the x-ray assembly, the support structures of the x-ray assembly and of the slip ring assembly of the subject invention are integrated and supported by a single hearing assembly. The resultant structure thus has an open bore and is concentric with the position of the patient's body, and provides for a substantial amount of gantry tilt and substantial patient access. The arrangement shown is compatible with fourth generation CT scanner gantries, and the combination of using a known fourth generation nutation approach in conjunction with the integrated slip ring of the subject invention provides for a very compact gantry design in comparison to conventional "black box" slip ring approaches.

The subject invention further incorporates an integrated arrangement of x-ray optics in which all of the optics (i.e. shutter, collimator, wedges, etc) are aligned in one fixture which may be mounted away from the main gantry structure. This integration makes it more convenient to use an optical bench for optical device assembly and alignment rather than attempt ing assembly and optical alignment on a cumbersome scanner gantry. Previous fourth generation nutating scanners had been designed so that each optical device is independently mounted on the gantry, which requires independent alignment of each device on the gantry. The preferred form of the present invention, accordingly, provides a significant advance over such prior art arrangements.

The incorporation of an integrated slip ring configuration also permits the same large diameter ring bearings to support the x-ray optics and slip ring rotor, and permits a slip ring geometry which allows the outline of an x-ray source envelope to be shaped for efficient utilisation of space. The slip ring stator casting has a second function as the gantry structural main frame which supports the rotor, rotor drive motor, and gantry covers and the like.

Separate anode and cathode chambers which are offset from one another minimise the possibility of high voltage breakdown. Moreover, the slip ring rotor casting has a second function, namely the structural support for x-ray optics, a heat exchanger, counterweights, and control electronics. The rotor casting may also act as a timing belt pulley for rotating the x-ray optics.

The rotor and stator castings of the subject invention act as Faraday shields.

The conical geometry of the slip ring assembly allows for a large internal diameter tunnel capable of substantial gantry tilting. This large diameter tunnel permits comfortable patient positioning even with 30° gantry tilting.

Replacement of both slip ring seals can be accomplished without separation of the rotor and stator sections.

It is also possible to rotate the brush assemblies with the rotor while maintaining the stationary slip ring attached to the stator which is the reverse of that shown in Figure 1.

## Claims

1. An integrated gantry assembly for an x-ray scanner comprising:

a stator (10) having an axial bore of sufficient size to encircle a patient positioned along the axis (14);

a rotor (16) having an axial bore of sufficient size to encircle a patient positioned along said axis;

a bearing assembly (18) located between said stator (10) and said rotor (16) so as to align said stator (10) and rotor (16) coaxially and to support said rotor (10) rotatably on said stator (10);

slip ring means (44 and 48) mounted in part on said stator (10) and in part on said rotor (16) and located between said rotor (16) and stator (10) for effecting high voltage connection from said stator (10) to said rotor (16) while permitting continuous rotation of said rotor (16); and

X-ray means (74) mounted onto and supported solely by said rotor (16) and defining an exposure position for said patient; characterised in that:

said stator (10) and rotor (16) define between them first and second annular recesses (36 and 38), said first recess (36) being positioned axially closer to said exposure position and having a smaller diameter than said second recess (38), one of said recesses (36 and 38) housing a negative electrode (44 or 48) of said slip ring (44 and 48) means and the other of said recess (36 or 38) housing a positive electrode (44 or 48) of said slip ring means (44 and 48).

2. An integrated gantry assembly as claimed in Claim 1, characterised in that the inside surface of said stator (10) which faces said patient is of a truncated conical shape with its smaller diameter portion being positioned axially closer to said exposure position.

3. An integrated gantry assembly as claimed in Claim 1 or 2, characterised in that the longitudinal cross section of said stator (10) and rotor (16) includes a series of matching steps, with the horizontal portions of at least two of the steps of said stator (10) separated from respective aligned horizontal portions of said steps of said rotor (16) to form two electrically and physically separated recesses (36 and 38) therebetween, with at least a portion of said slip ring means (44 and 48) located in each of said recesses (36 and 38).

4. An integrated gantry assembly as claimed in Claim 3, characterised in that first and second seals (62 and 64) are pancaked between said steps of said rotor (16) and stator (10), with one seal (62 or 64) located on each side of said recesses (36 and 38).

5. An integrated gantry assembly as claimed in Claim 4, characterised in that said seals (62 and 64) are each located by a respective seal retainer (66 or 68), and said seal retainers (66 and 68) are adjustably positionable to pre-load said seals (62 and 64).

6. An integrated gantry assembly as claimed in Claim 3 characterised by means for making electrical connections (52 and 56) to said recesses (36 and 38) through vertical portions of said steps of said stator (10) which define said recesses (36 and 38), and that the axes of said electrical connection means (52 and 56) are parallel to said longitudinal axes of said rotor (16) and stator (10).

7. A gantry assembly including a stator and a rotor in which

(a) the stator comprises:

i. first and second cylindrical sections of different diameters which are axially offset from one another along the axis of rotation, said first section having a smaller diameter than said second section and having a diameter sufficient to encircle a patient positioned along said axis of rotation; and

ii. first and second annular plate sections lying in a plane perpendicular to said axis of rotation, said first plate section extending from one edge of the smaller of said cylindrical sections and the corresponding edge of the larger of said cylindrical sections, and said second plate section extending from the other edge of said second cylindrical section in a direction away from said axis of rotation;

(b) the rotor comprises;

i. first and second cylindrical sections aligned with and coaxial to said first and second cylindrical sections of said stator, respectively, the diameters of said first and second cylindrical sections of said rotor being greater than the diameters of said first and second cylindrical sections of said stator respectively, to form respective first and second annular recesses therebetween with said first annular plate section of said stator closing off one side of said first recess and said second plate section of said stator closing off one side of said second recess; and

ii. first and second annular plate sections lying in planes perpendicular to said axis of rotation, said first annular plate section extending from one side of said first cylindrical section of said rotor to close off the other side of said first recess and said second annular plate section extending from the other edge of said first cylindrical section of said rotor to one edge of said second cylindrical section of said rotor to close off the other side of said recess;

the assembly further comprising;

(c) a bearing assembly located between said stator and said rotor to rotatably support said rotor on said stator; and

(d) first and second slip ring means mounted in part on said stator and in part on said rotor and extending into said first and second recesses, respectively, for effecting high voltage connection from said stator to said rotor while permitting continuous rotation of said rotor.

8. A gantry assembly according to Claim 7 including x-ray means mounted onto and supported solely by said rotor and defining an exposure position which is axially closer to said first recess than said second recess.

9. An integrated gantry assembly according to Claim 7 wherein first and second pancake electrodes are respectively mounted in said first and second recesses on said first and second annular plate sections of said rotor.

10. An integrated gantry assembly according to Claim 7 including positive and negative high voltage connectors one of which extends through said first annular plate section of said stator into said first recess and the other of which extends through said second annular plate section of said stator into said second recess, the axes of said connectors lying parallel to said axis of rotation of said assembly.

11. An integrated gantry assembly according to Claim 7 wherein said x-ray means is mounted onto the outside surface of at least one of said first annular plate section and said first cylindrical section of said rotor, and said x-ray means extends in part and overlaps said first cylindrical section of said rotor.

12. An integrated gantry assembly according to any one of Claims 1 to 6 or Claim 9 or Claim 10 wherein said x-ray means comprises;

(a) a bracket removably attached to said rotor;

(b) an x-ray source attached to said bracket; and

(c) X-ray optics attached to said bracket, whereby said x-ray source and x-ray optics can be aligned on said bracket remote from said rotor and stator.

**Patentansprüche**

1. Integrierte Gehäuseeinheit für ein Röntgenabtastgerät mit

einem Stator (10), der eine axiale Bohrung ausreichender Größe aufweist zur Aufnahme eines entlang der Achse (10) positionierten Patienten,

einem Rotor (16), der eine axiale Bohrung ausreichender Größe zu Umgebung eines entlang der Achse positionierten Patienten aufweist,

einer Lagereinheit (18), die zwischen dem Stator (10) und dem Rotor (16) angeordnet ist, um den Stator (10) und den Rotor (16) koaxial auszurichten und um den Rotor (10) drehbar auf dem Stator (10) zu lagern,

einer Schleifringanordnung (44 und 48), die teilweise auf dem Stator (10) und teilweise auf dem Rotor (16) angebracht und zwischen Rotor (16) und Stator (10) angeordnet ist, um eine Hochspannungsverbindung vom Stator (10) zum Rotor (16) herzustellen unter Zulassung einer kontinuierlichen Drehung des Rotors (16), und

einer Röntgeneinrichtung (74), die nur auf dem Rotor (16) angeordnet ist und eine Belichtungsposition für den Patienten definiert, dadurch gekennzeichnet, daß

zwischen dem Stator (10) und dem Rotor (16) eine erste und eine zweite ringfömige Ausnehmung (36 und 38) vorgesehen sind, wobei die erste Ausnehmung (36) axial näher an der Belichtungsposition angeordnet ist und einen geringeren Durchmesser aufweist als die zweite Ausnehmung (38), wobei eine der Ausnehmungen (36 und 38) eine negative Elektrode (44 oder 48) des Schleifringes (44 und 48) aufnimmt und die andere der Ausnehmungen (36 oder 38) eine positive Elektrode (44 oder 48) des Schleifringes (44 und 48) aufnimmt.

2. Integrierte Gehäuseeinheit nach Anspruch 1, dadurch gekennzeichnet, daß die Innenfläche des Stators (10), die zum Patienten zeigt, eine abgerundete konische Form aufweist, deren Teil mit dem geringeren Durchmesser axial näher zur Belichtungsposition angeordnet ist.

3. Integrierte Gehäuseeinheit nach Anspruch 1 oder 2, dadurch gekennzeichnet, daß der Längsschnitt des Stators (10) und des Rotors (16) eine Reihe zusammenpassender Stufen aufweist, wobei die horizontalen Teile wenigstens zweier Stufen des Stators (10) von entsprechend ausgerichteten Teilen der Stufen des Rotors (16) getrennt sind, um dazwischen zwei elektrisch und physikalisch getrennte Ausnehmungen (36 und 38) zu bilden, wobei wenigstens ein Teil des Schleifringes (44 und 48) in jedem der Ausnehmungen (36 und 38) angeordnet ist.

4. Integrierte Gehäuseeinheit nach Anspruch 3, dadurch gekennzeichnet, daß die erste und zweite Dichtung (62 und 64) zwischen den Stufen des Rotors (16) und des Stators (10) angeordnet sind, wobei eine Dichtung (62 oder 64) auf jeder Seite der Ausnehmungen (36 und 38) angeordnet ist.

5. Integrierte Gehäuseeinheit nach Anspruch 4, dadurch gekennzeichnet, daß die Dichtungen (62 und 64) jeweils mittels eines entsprechenden Dichtungshalters (66 oder 68) angeordnet sind und daß die Dichtungshalter (66 und 68) zum Vorspannen der Dichtungen (62 und 64) einstellbar positionierbar sind.

6. Integrierte Gehäuseeinheit nach Anspruch 3, gekennzeichnet durch Mittel zur Herstellung elektrischer Verbindungen (52 und 56) mit den Ausnehmungen (36 und 38) über senkrechte Teile der Stufen des Stators (10), die die Ausnehmungen (36 und 38) definierten, wobei die Achsen der elektrischen Verbindungsmittel (52 und 56) parallel zur Längsachse des Rotors (16) und des Stators (10) verlaufen.

7. Gehäuseeinheit mit einem Stator und einem Rotor, bei der

a) der Stator

i) einen ersten und einen zweiten zylindrischen Abschnitt mit unterschiedlichen Durchmessern aufweist, die axial entlang der Drehzahl versetzt zueinander angeordnet sind, wobei der erste Abschnitt einen Durchmesser aufweist, der geringer ist als der des zweiten Abschnittes und der ausreicht, einen entlang der Drehachse positionierten Patienten zu umgeben, und

ii) einen ersten und einen zweiten ringförmigen Plattenabschnitt umfaßt, die in einer senkrecht zur Drechachse liegenden Ebene angeordnet sind, wobei der erste Plattenabschnitt sich von einer Kante des kleineren zylindrischen Abschnittes und der entsprechenden Kante des größeren zylindrischen Abschnittes aus erstreckt und sind der zweite plattenförmige Abschnitt von der anderen Kante des zweitenzylindrischen Abschnittes in einer von der Drehachse wegführenden Richtung erstreckt,

b) der Rotor

i) einen ersten und einen zweiten zylindrischen Abschnitt aufweist, die entsprechend mit einem zu dem ersten und zweiten zylindrischen Abschnitt des Stators koaxialen Abschnitt ausgerichtet sind, wobei die Durchmesser des ersten und zweiten zylindrischen Abschnittes des Rotors größer sind als die Durchmesser des ersten und zweiten zylindrischen Abschnittes des Stators, um dazwischen entsprechend eine erste und eine zweite ringförmige Ausnehmung zu bilden, wobei der erste ringförmige Plattenabschnitt des Stators eine Seite der ersten Ausnehmung und der zweite Plattenförmige Abschnitt des Stators eine Seite der zweiten Ausnehmung abschließt, und

ii) einen ersten und einen zweiten ringförmigen Plattenabschnitt aufweist, die in senkrecht zur Drehachse liegenden Ebenen angeordnet sind, wobei der erste ringförmige Plattenabschnitt sich von einer Seite des ersten zylindrischen Abschnittes des Rotors erstreckt zum Abschließen der anderen Seite der ersten Ausnehmung und sich der zweite ringförmige Plattenabschnitt von der anderen Kante des ersten zylindrischen Abschnittes des Rotors zu einer Kante des zweiten zylindrischen Abschnittes des Rotors erstreckt, um die andere Seite der Ausnehmung abzuschließen, wobei die Einheit ferner

c) eine Lageranordnung aufweist, die zwischen dem Stator und dem Rotor zur drehbaren Lagerung des Rotors auf dem Stator angeordnet ist und

d) einen ersten und einen zweiten Schleifring umfaßt, die teilweise auf dem Stator und teilweise auf dem Rotor angebracht sind und sich in die erste und zweite Ausnehmung erstrecken zur Herstellung einer Hochspannungsverbindung

vom Stator zum Rotor unter Zulassung einer stetigen Drehung des Rotors.

8. Gehäuseeinheit nach Anspruch 7 mit einer Röntgenstrahleinrichtung, die auf dem Rotor angebracht ist und lediglich vom Rotor getragen wird und eine Belichtungsposition definiert, die axial näher zur ersten Ausnehmung als zur zweiten Ausnehmung angeordnet ist.

9. Gehäuseeinheit nach Anspruch 7, bei der eine erste und eine zweite Schreibenelektrode entsprechend in der ersten und zweiten Ausnehmung auf dem ersten und zweiten ringförmigen Plattenabschnitt des Rotors angeordnet sind.

10. Gehäuseeinheit nach Anspruch 7 mit positiven und negativen Hochspannungsverbindungsmitteln, von denen sich ein Verbindungsmittel durch den ersten ringförmigen Plattenabschnitt des Stators in die erste Ausnehmung und sich die andere durch den zweiten ringförmigen Plattenabschnitt des Stators in die zweite Ausnehmung erstrekt, wobei die Achsen der Verbindungsmittel parallel zur Drehachse der Anordnung angeordnet sind.

11. Gehäuseeinheit nach Anspruch 7, bei der die Röntgenstrahleinrichtung auf der Außenfläche wenigstens des ersten ringförmigen Plattenabschnittes oder des ersten zylindrischen Abschnittes des Rotors angeordnet ist und sich teilweise in den ersten zylindrischen Abschnitt des Rotors erstreckt und diesen überlappt.

12. Gehäuseeinheit nach einem der Ansprüche 1 bis 6 oder 9 oder 10, bei der die Röntgenstrahleinrichtung

a) einen lösbar auf dem Rotor angeordneten Sockel,

b) eine auf dem Sockel befestigte Röntgenstrahlquelle, und

c) eine auf dem Sockel angebrachte Röntgenstrahloptik aufweist, wobei die Röntgenstrahlquelle und die Röntgenstrahloptik entfernt vom Rotor und Stator auf dem Sockel ausgerichtet werden können.

**Revendications**

1. Dispositif de portique intégré pour un scanner à rayons X qui comprend:

un stator (10) présentant un trou axial dont les dimensions sont suffisantes pour encercler le patient placé le long de l'axe (14);

un rotor (16) ayant une ouverture axiale de dimensions suffisantes pour encercler le patient placé le long dudit axe;

un ensemble de paliers (18) placée entre ledit stator (10) et ledit rotor (16) afin d'aligner ledit stator (10) et rotor (16) coaxialement et pour supporter ledit rotor (16) à rotation sur ledit stator (10);

des bagues collectrices (44 et 46) montées en partie sur ledit stator (10) et en partie sur ledit rotor (16) et interposées entre ledit rotor (16) et ledit stator (10) afin d'établir une connexion de haute tension entre ledit stator (10) et ledit rotor (16), tout en permettant une rotation continue dudit rotor (16); et

une source de rayons X (74) montée sur et supportée uniquement par ledit rotor (16) et qui définit une position d'exposition pour ledit patient, caractérisé en ce que:

ledit stator (10) et ledit' rotor (16) définissent entre eux deux cavités annulaires (36 et 38), la première cavité (36) étant située axialement plus près de ladite position d'expositionet ayant un plus petit diamètre que la seconde cavité (38), l'une de ces deux cavités (36 et 38) logeant une électrode négative desdites bagues collectrices (44 et 48), tandis que l'autre desdites cavités (36 ou 38) loge un électrode positive desdites bagues collectrices (44 et 48).

2. Dispositif de portique intégré selon la revendication 1, caractérisé en ce que la surface intérieure dudit stator (10) qui fait face au patient à la forme d'un cône tronqué dont la partie de plus petit diamètre est placée axialement plus près de ladite position d'exposition.

3. Dispositif de portique intégré selon la revendication 1 ou 2, caractérisé en ce que la section longitudinale dudit stator (10) et dudit rotor (16) présente une série de gradins, les parties horizontales, d'au moins deux des gradins dudit stator (10) étant séparés des parties horizontales alignées respectives des gradins dudit rotor (16) de manière à former deux renfoncements séparés physiquement et électriquement (36 et 38) entre eux, une partie, au moins, desdites bagues collectrices (44 et 48) étant logée dans chacun desdits renfoncements (36, 38).

4. Dispositif de portique intégré selon la revendication 3, caractérisé en ce que deux joints (62 et 64) sont disposés entre lesdits gradins lesdits rotors (16) et stators (10), l'un de ces joints (62 ou 64) étant placé de chaque côté desdits renfoncements (36 et 38).

5. Dispositif de portique intégré selon la revendication 4, caractérisé en ce que chacun desdits joints (62 et 64) est tenu en position par un frein (66 ou 68), et en ce que lesdits freins (66 et 68) peuvent être placés et préréglés afin de précharger lesdits joints (62 et 64).

6. Dispositif de portique intégré selon la revendication 3, caractérisé par des moyens pour établir des connexions électriques (52 et 54) avec lesdites cavités (36 et 38) par des parties verticales desdits gradins dudit stator (10) qui définissent lesdits renfoncements (36 et 38), et en ce que les axes desdites connexions électriques (52 et 54) sont parallèles aux axes longitudinaux desdits rotors (16) et stator (10).

7. Dispositif de portique comportant un stator et un rotor dans lequel:

a) le stator comprend:

I. une première et une seconde sections cylindriques des différents diamères qui sont axialement décalés le long.de l'axe de rotation, ladite première section ayant un plus petit diamètre que la seconde, tout en ayant un diamètre suffisant pour encercler le patient placé le long dudit axe de rotation; et

II. une première et une seconde section de plaque annulaire situées dans un plan perpendiculaire audit aux de rotation, la première section s'étendant de l'un des bords de la plus petite desdites sections cylindriques et du bord correspondant de la plus grande desdites sections cylindriques, tandis que la seconde section de plaque s'étend de l'autre bord de la seconde section cylindrique dans une direction opposée audit axe de rotation;

b) le rotor comprend:

I. un première et une seconde sections respectivement alignées et coaxiales auxdites première et seconde sections cylindriques dudit stator, les diamètres de ces deux sections cylindriques dudit rotor étant plus grands que les diamètres des deux sections cylindriques dudit stator afin de former des premiers et des seconds renfoncements annulaires entre eux, la disposition étant telle que ladite première section de plaque annulaire dudit stator ferme l'un des côtés dudit premier renfoncement, tandis que la seconde section de plaque dudit stator ferme l'un des côtés du second renfoncements; et

II. les premières et secondes sections de plaque annulaire étant situées dans des plans perpendiculaires audit axe de rotation d'un côté de ladite première section cylindrirque dudit rotor de manière à fermer l'autre côté du premier renfoncement, tandis que la second section de plaque annulaire s'étend de l'autre bord de ladite première section cylindrique dudit rotor à l'un des bords de la seconde section cylindrique dudit rotor de manière à fermer l'autre côté dudit renfoncement;

le dispositif comprenant, en outre:

c) un ensemble de paliers disposés entre ledit stator et ledit rotor afin de supporter à rotation ledit rotor sur ledit stator; et

d) un première et une seconde bagues collectrices montées en partie dans ledit stator et en partie dans ledit rotor et s'étendant respectivement dans lesdits premier et second renfoncements afin d'établir une connexion à haute tension entre ledit stator et ledit rotor pour permettre une rotation continue de ce dernier.

8. Dispositif de portique selon la revendication 7, caractérisé en ce qu'il comprend une source de rayons X montée sur et supportée uniquement par ledit rotor et définissant une position d'exposition qui est axialement plus proche dudit premier renfoncements que du second.

9. Dispositif de portique selon la revendication 7, caractérisé en ce qu'une première et une seconde électrodes sont respectivement montées sur lesdits première et seconde sections de plaque annulaire dudit rotor.

10. Dispositif de portique intégré selon la revendication 7, caractérisé en ce qu'il comporte des connecteurs de haute tension positifs et négatifs dont l'un s'étend à travers ladite première section de plaque annulaire dudit stator dans ledit premier renfoncement, tandis que l'autre s'étend à travers ladite seconde section de plaque annulaire dudit stator dans ledit second renfoncement, les axes desdits connecteurs s'étendant parallèlement à l'axe de rotation du dispositif.

11. Dispositif de portique intégré selon la revendication 7, caractérisé en ce que ladite source de rayons X est montée sur la surface extérieure soit de la première section de plaque annulaire, soit de ladite première section cylindrique dudit rotor, ladite source de rayons X s'étendant en partie dans et au-dessus de ladite première section cylindrique dudit rotor.

12. Dispositif de portique intégré selon l'une quelconque des revendications 1 à ou 9 à 10, caractérisé en ce que ladite source de rayons X comprend:

a) un support fixé de façon amovible audit rotor;

b) une source de rayons X fixée audit support; et

c) une optique pour rayons X fixée audit support, ce qui fait que ladite source de rayons X et ladite optique à rayons X peuvent être alignés sur ledit support à une certaine distance dudit rotor et dudit stator.

Fig.I.

EP 0 125 879 B1

Fig.2.